(19) Europäisches Patentamt / European Patent Office / Office européen des brevets

(11) **EP 2 346 486 B1**

(12) **FASCICULE DE BREVET EUROPEEN**

(45) Date de publication et mention
de la délivrance du brevet:
**10.10.2018 Bulletin 2018/41**

(21) Numéro de dépôt: **09748788.8**

(22) Date de dépôt: **09.11.2009**

(51) Int Cl.:
*A61K 8/63* *(2006.01)*    *A61K 8/64* *(2006.01)*
*A61K 8/73* *(2006.01)*    *A61Q 19/00* *(2006.01)*
*A61K 31/715* *(2006.01)*

(86) Numéro de dépôt international:
**PCT/EP2009/064860**

(87) Numéro de publication internationale:
**WO 2010/052327 (14.05.2010 Gazette 2010/19)**

(54) **NOUVEL ACTIF ANTI-VERGETURES ET COMPOSITIONS LE COMPRENANT**

NEUER WIRKSTOFF GEGEN DEHNUNGSSTREIFEN UND DIESEN ENTHALTENDE
ZUSAMMENSETZUNGEN

NOVEL ANTI-STRETCH MARK ACTIVE AGENT, AND COMPOSITIONS CONTAINING SAME

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **07.11.2008  FR 0857579**

(43) Date de publication de la demande:
**27.07.2011  Bulletin 2011/30**

(73) Titulaire: **Laboratoires Expanscience
92048 Paris La Défense Cedex (FR)**

(72) Inventeurs:
• **MSIKA, Philippe
F-78000 Versailles (FR)**
• **BAUDOUIN, Caroline
F-78120 Rambouillet (FR)**
• **NAAIMI, Dalale
F-28230 Epernon (FR)**
• **MENU, Franck
F-28260 Sorel Moussel (FR)**

(74) Mandataire: **Regimbeau
87 rue de Sèze
69477 Lyon Cedex 06 (FR)**

(56) Documents cités:
**EP-A- 0 668 072      WO-A-2007/122422
WO-A-2008/080443    DE-A1-102005 007 482**

**Description**

La présente invention est relative à un nouvel actif anti-vergeture, l'arabinogalactane, et à des compositions cosmétiques le comprenant.

INTRODUCTION

Les vergetures (striae distensae) sont des stries cutanées qui apparaissent suite à une distension exagérée (gain de poids) de la peau et à une modification hormonale. Elles forment des stries cutanées parallèles et longilignes, de 5 à 15 centimètres de longueur et de 0,2 à 1 centimètre de largeur. Elles peuvent être fines et peu apparentes, mais elles présentent parfois de petites dépressions qui donnent à la peau un aspect fripé. Les vergetures varient au début du rose pâle au rouge violacé (vergetures immatures ou inflammatoires). Elles tendent à changer de couleur au cours du temps et à prendre un aspect blanc nacré (vergetures matures). Elles deviennent alors moins apparentes, mais la cicatrice demeure. Les vergetures concernent le plus souvent les femmes pendant la puberté ou lors d'une grossesse. Environ 50% à 70% de femmes enceintes vont développer des vergetures. Elles apparaissent généralement entre le sixième et le huitième mois. Les femmes très jeunes développent plus fréquemment des vergetures (20% des adolescentes ont des vergetures sévères) et certains facteurs les favorisent comme la première grossesse, les grossesses multiples et une prise de poids rapide et excessive (plus de 15 kg). Les facteurs génétiques ainsi que l'hérédité exercent également une influence sur leurs apparitions. Les vergetures liées à la grossesse siégent surtout sur la face antérieure de l'abdomen, mais existent aussi au niveau des seins, des cuisses et des hanches.

Les vergetures peuvent également apparaître lors d'états physiologiques ou pathologiques tels que l'obésité, un régime alimentaire relativement intensif, la tuberculose et la fièvre typhoïde. Elles peuvent également être signe de certaines maladies génétiques rares comme le syndrome de Prader-Willi, le syndrome de Laurence-Moon Biedel et le syndrome d'Ehlers-Danlos. Elles peuvent aussi révéler une surproduction de cortisol par les glandes surrénales que l'on appelle le syndrome de Cushing. Ce syndrome peut être causé par une tumeur de l'hypophyse ou des glandes surrénales ou par un usage prolongé de corticoïdes utilisés dans le traitement de certaines maladies comme l'asthme.

Les vergetures naissent sous l'effet d'un étirement trop rapide et brutal de la peau. Chaque vergeture ressemble à une déchirure de la peau. C'est le tissu dermique qui est en réalité altéré, et la cellule cible de ces atteintes est le fibroblaste. Le fibroblaste subit un changement phénotypique en se transformant en myofibroblaste sous l'influence des distensions mécaniques, et voit son métabolisme altéré sous l'effet de l'environnement hormonal. En effet, lors de la grossesse, l'augmentation des hormones conduit à une élévation naturelle des corticoïdes, qui inhibent l'activité des fibroblastes par une action anti-mitotique. Ainsi, le métabolisme de ces cellules est ralenti. De plus, les corticoïdes agissent sur le métabolisme du tissu conjonctif en limitant la synthèse de collagène et d'élastine, tout en activant leur dégradation. Lors d'une grossesse, la peau est donc particulièrement fragilisée. Par ailleurs, ce processus s'accompagne d'une phase inflammatoire. En conséquence, il y a dégénérescence du tissu dermique conduisant à la formation d'une cicatrice dermique atrophique.

Les principaux facteurs déclenchant sont donc l'inflammation, le stress mécanique et l'environnement hormonal. L'ensemble de ces facteurs provoque au final un étirement, une désorientation, une désorganisation des fibres de collagène et d'élastine, sans rupture du tissu de soutien. La cause majeure de la rupture élastique provient également d'un manque d'eau dans les cellules. Dès les premiers signes de déshydratation, les fibres du collagène forment des ramifications nombreuses et rapprochées qui, à force de tension, perdent de leur élasticité et se cassent de façon visible au niveau du derme.

Ainsi, dans une vergeture récente, qui apparaît comme une strie rouge-violacée, il existe une inflammation qui désorganise et détruit les fibres de la peau. Petit à petit, l'inflammation et la rougeur diminuent et les fibroblastes tentent de synthétiser de nouvelles fibres pour combler le vide, ces nouvelles fibres étant de moins bonnes qualité que les anciennes. Quelques mois plus tard, la vergeture ancienne prend une couleur blanc-nacré et un aspect brillant.

Les vergetures sont assimilables à des cicatrices (car elles ont subi les mêmes étapes de formation qu'après un traumatisme de la peau), leur guérison est actuellement impossible, mais une atténuation et une amélioration des lésions sont possibles. Les traitements curatifs sont essentiellement locaux : emploi de topiques avec de la trétinoïne (all-trans acide rétinoïque) ou des acides de fruits, emploi de peeling ou de laser. La trétinoine aurait une action anti-vergetures tendant à restaurer essentiellement le réseau des fibrillines.

Compte tenu de la physiopathologie de la vergeture, la prévention et la réduction des vergetures naissantes liées à la grossesse nécessite l'utilisation d'un produit ciblant le fibroblaste dermique, pouvant agir en activant la prolifération cellulaire et en stimulant le métabolisme en particulier la néo synthèse des principales macromolécules responsables de l'élasticité et la tonicité de la peau (collagènes et élastine). D'autre part, il faut également limiter l'inflammation qui participe à la dégradation des macromolécules de la matrice dermique.

## ART ANTERIEUR

La demande WO 00/19974 décrit une méthode cosmétique de prévention de l'apparition et/ou de traitement des vergetures. La composition cosmétique proposée comprend au moins un agent anti-vergetures choisi parmi des peptides de soja, des tripeptides constitués des acides aminés glycine, histidine et lysine, et les mélanges de ces peptides.

Si cette composition de l'art antérieur permet d'obtenir un effet de régression des vergetures, elle n'est pas totalement adaptée au cas particulier des vergetures apparaissant pendant la grossesse, et notamment pour la prévention de leur apparition.

Il existe une réelle demande pour l'élaboration d'un produit permettant de prévenir et/ou de traiter efficacement, avec une tolérance cutanée acceptable, ce phénomène complexe et particulièrement inesthétique que sont les vergetures apparaissant au cours de la grossesse.

Les arabinogalactanes (aussi appelé galactoarabinanes) sont des polysaccharides. Elles sont présentes en quantités variables dans de multiples végétaux, champignons et bactéries. Les arabinogalactanes sont des fibres solubles naturelles qui peuvent être extraites à partir de bactéries ou de plantes telles que le café ou les mélèzes.

L'arabinogalactane est un polymère composé de deux types de saccharides, le galactose et l'arabinose, dans un ratio de 6 :1 respectivement.

Des méthodes d'extraction de l'arabinogalactane ont été décrites, en particulier à partir de café (EP 1 600 461, WO 2007/099997) et de mélèze (EP 0 866 808). Dans le commerce, les arabinogalactanes les plus courantes sont celles issues de mélèze, un arbre particulièrement riche en arabinogalactanes.

Les rôles de l'arabinogalactane sur le métabolisme des mammifères connus à ce jour sont les suivants :

1- Les arabinogalactanes sont des fibres non digestibles qui favorisent la prolifération, dans le tube digestif, de bactéries utiles à l'organisme. C'est ce qu'on appelle une action « prébiotique ».

2- L'ingestion d'arabinogalactane a la réputation de stimuler le système immunitaire. La demande EP 1 600 461 revendique en particulier l'ajout d'arabinogalactane issu de grains de café dans des aliments afin d'obtenir des « aliments santé ».

Il existe toutes sortes de dérivés d'arabinogalactane. Ces dérivés ont des applications cosmétiques et dermatologiques telles que décrites dans les documents suivants :

- Le brevet EP 0 939 771 décrit des compositions comprenant des dérivés lipidiques de l'arabinogalactane. La demande EP 1 076 547 décrit une composition comprenant un dérivé d'arabinogalactane, et son utilisation en cosmétique.
- Les protéines couplées à des arabinogalactanes (AGP) ont été largement étudiées, et leur rôle en cosmétique a été décrit notamment dans le brevet EP 0 668 072.

L'utilisation d'arabinogalactane pur en cosmétique a été proposée dans la demande FR2836378, pour son action protectrice et stimulatrice sur l'interleukine-12. Cette utilisation est destinée à favoriser la réparation de l'ADN.

De manière tout à fait surprenante, les inventeurs de la présente demande ont mis en évidence un effet bénéfique de l'arabinogalactane sur le traitement et la prévention des vergetures.

## DESCRIPTION DETAILLEE DE L'INVENTION

La présente invention décrit une méthode de prévention et/ou de traitement cosmétique des vergetures de la peau, caractérisée par le fait que l'on administre à une personne susceptible de former ou ayant des vergetures, une composition comprenant en tant que principe actif de l'arabinogalactane. Cette composition peut être avantageusement administrée par voie topique ou par voie orale.

De par ses propriétés non irritantes et hydratantes, l'arabinogalactane permet de réduire significativement la perte insensible en eau. Par ailleurs, ce polysaccharide possède une capacité d'exfoliation intrinsèque. Ces actions combinées favorisent le renouvellement cellulaire.

Par l'expression « prévention des vergetures de la peau », on entend une action permettant d'éviter ou à tout le moins de réduire la formation de vergetures, c'est-à-dire leur longueur, largeur et/ou profondeur, dans le cadre d'un traitement cosmétique ou dermatologique, par application de la composition, avant et au cours d'un évènement connu comme pouvant provoquer l'apparition de vergetures, tel qu'une grossesse.

Par l'expression « traitement des vergetures de la peau » on entend une action permettant de faire régresser, c'est-à-dire de résorber, dans le cadre d'un traitement cosmétique ou dermatologique, de manière visible et mesurable, de vergetures déjà formées, c'est-à-dire leur longueur, largeur et/ou profondeur.

Ainsi, la composition utilisée selon l'invention peut être appliquée par voie topique sur des zones de la peau susceptibles de former des vergetures, comprenant des vergetures en cours de formation ou même comprenant des vergetures déjà formées. Ces zones sont bien connues de l'homme du métier, et correspondent à la zone du ventre, des hanches, des fesses, des cuisses et des seins.

La composition peut également être administrée par voie orale, notamment sous forme de comprimés, gélules, capsules molles, dragées, émulsions, gels, ou sous forme de compléments ou produits alimentaires.

Selon un aspect préféré de l'invention, l'arabinogalactane utilisée est extraite de mélèze. Un procédé d'extraction est décrit en particulier dans la demande de brevet EP 0 866 808.

Préférentiellement, la composition anti-vergetures selon l'invention comprend de l'arabinogalactane dans une proportion comprise entre 0,01% et 10% en poids, avantageusement entre 1 et 5%, et plus préférentiellement dans une proportion d'environ 2% en poids par rapport au poids total de la composition.

Selon un aspect préféré de l'invention, la composition anti-vergetures comprend au moins un autre agent anti-vergetures, et en particulier :

- du lupéol,
- des peptides de soja,
- des tripeptides constitués des acides aminés Glycine, Histidine et Lysine,
- un extrait de fleur de sophora (*Sophora Japonica),*
- un extrait de chlorophycée (*enteromorpha compressa)*
- un extrait peptidique d'avocat,
- du panthénol,
- ainsi que les divers mélanges de ces composés.

Chacun de ces composés à une action propre visant à amplifier ou favoriser l'action de l'arabinogalactane sur les vergetures.

Le lupéol est un alcool triterpénique, présent dans de nombreux végétaux. La composition de lupéol préférée est composée de 5% d'extrait de lupin (*Lupinus Albus*) et de 95% d'huile de pépins *d'Helianthus Annuus.* Un extrait de coques de graines de lupin contenant du lupéol peut être préparé tel que décrit dans la demande WO 02/085827. Le lupéol favorise la production de « bon collagène » par les cellules, et agit sur les fibroblastes en les « déstressant » ; la peau est mieux préparée pour affronter des contraintes mécaniques.

Les « peptides de soja » selon la présente invention peuvent être tout peptide obtenu par hydrolyse de protéines extraites du soja, selon des conditions opératoires connues de l'homme du métier, en d'autres termes tout hydrolysat de protéine du soja. Les peptides de soja ont une action d'élasto-régulateur, permettant de promouvoir l'élasticité de la peau.

Un peptide du soja particulièrement préféré pour la composition utilisée selon l'invention est le peptide fermenté dénommé « Phytokine® » tel que commercialisé par la société COLETICA.

Par « tripeptides constitués des acides aminés Glycine, Histidine et Lysine », on entend en particulier les tripeptides de séquence Gly-His-Lys , dont les acides aminés peuvent être sous la forme D, L ou DL, éventuellement conjugués avec un acide carboxylique tel que l'acide acétique, sous forme d'un complexe avec un métal tel que le zinc ou le cuivre.

L'extrait de fleur de *Sophora Japonica* est riche en flavonoïdes et rutine. Cet extrait peut désigner notamment une composition contenant 22,5% d'extrait de fleurs, 55% de butylène glycol et 22,5% d'eau. C'est un composé contribuant de manière secondaire à l'action anti-vergetures, qui participe à un contrôle de la vascularisation des vergetures et donc de la couleur de ces dernières.

Par « un extrait de chlorophycée » on entend notamment un extrait *d'enteromorpha compressa.* Avantageusement, on utilisera une composition comprenant 20% *d'enteromorpha compressa* contenu dans un mélange de butylène glycol (40%) et de glycérine (40%). Ce composé a une action apaisante, de réduction de la desquamation, de la sensation de tiraillement de la peau et de l'érythème. Il permet l'amélioration du confort des peaux fines, sensibles et sèches.

Par « extrait peptidique d'avocat » on entend notamment un extrait tel que décrit dans la demande internationale WO 2005/105123, cet extrait étant caractérisé en ce qu'il comprend 2 à 10% en poids d'azote alpha-aminé par rapport au poids de la matière sèche de l'extrait peptidique. Cet extrait peptidique peut directement être obtenu à partir de n'importe quelle partie de l'avocat ou de l'avocatier, telle que le fruit, la peau, le noyau, la feuille ou les racines de l'avocatier. Les fruits pourront être choisis parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red,* plus avantageusement, les variétés *Hass, Fuerte et Reed.*

Le panthénol dextrogyre est une provitamine issue de l'acide D-pantothénique. Il stimule la formation des cellules, hydrate et apaise.

D'autres composés peuvent avantageusement être ajoutés à la composition selon l'invention, tels que le lactate méthysilanol ou des oligoéléments à base de cuivre et zinc, tels que le gluconate de zinc et le gluconate de cuivre.

La composition peut en outre comprendre au moins un composé choisi dans le groupe constitué par les agents anti-irritant et/ou apaisant et/ou cicatrisant et/ou anti-vieillissement et/ou hydratant.

Les agents anti-irritants et/ou apaisant et/ou cicatrisant et/ou anti-vieillissement et/ou hydratant pouvant être utilisés dans le cadre de la présente invention en association avec l'arabinogalactane sont avantageusement la glycine, les sucres et/ou peptides végétaux dont le lupin, le lupéol (FR 2 822 821, FR 2 857 596), les oxazolines (WO 04/112741, WO 2006/114443), les oxazolidinones (WO 04/05005), l'acide lipoïque, l'alpha bisabolol, les dérivés de réglisse, l'enoxolone, l'ectoïne, l'Avocadofurane® (furanes d'avocat, pouvant être obtenus par le procédé décrit dans la demande internationale WO 01/21605), les extraits de Centella asiatica en particulier l'acide madécassique ou l'acide asiatique, la caféine, le rétinol, le rétinal, l'acide rétinoïque, l'oxyde de zinc, le magnésium, le silicium, le cuivre, le zinc, le manganèse, le sélénium, l'acide hyaluronique, l'acide azélaïque et ses sels ou esters, l'acide salicylique et ses dérivés, l'alpha hydroxyacide (AHA), les esters d'AHA, l'acide pyrrolidone carboxylique et dérivés, les céramides, le cholesterol, le squalane, les phospholipides, le beta carotène, la vitamine A, la vitamine E, la vitamine C, la vitamine B3 (niacinamide, nicotinamide), la vitamine B5 (panthenol), la vitamine B6, le peroxyde de benzoyle, l'urée, la coenzyme Q10, la glucosamine et ses sels, la N-acétyl glucosamine, les eaux de source ou thermales (eau d'Avène, eau de la Roche Posay, eau de Saint Gervais, eau d'Uriage, eau de Gamarde), et les peptides de soja.

Les oxazolines pouvant être utilisées dans le cadre de la présente invention en association avec l'arabinogalactane sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

Outre ces actifs, l'arabinogalactane selon l'invention, seul ou en association avec les actifs précédemment cités, peut être utilisé en association avec les sucres d'avocat, des agents restructurant de la barrière cutanée, des composés antifongiques, des conservateurs antiseptiques et des composés contenant des insaponifiables d'huiles végétales, les peptides de lupin, l'huile d'avocat, le butyl avocadate, les cyclocéramides, la génistéine, les concentrats de colza, et les concentrats de maïs.

Les sucres d'avocats sont avantageusement le D-mannoheptulose et/ou le perséitol. Les sucres d'avocats correspondent plus avantageusement à l'extrait hydrosoluble de sucres d'avocat décrit dans la demande WO 2005/115421.

Les agents restructurant de la barrière cutanée, permettant de stimuler la synthèse des lipides clés de l'épiderme, et pouvant être utilisés dans le cadre de la présente invention en association avec l'arabinogalactane sont avantageusement des concentrats de tournesol, encore plus avantageusement des concentrats de tournesol linoléiques, tels que l'actif commercialisé par les Laboratoires Expanscience, Soline® (cf. la demande internationale WO 01/21150), des insaponifiables d'huile végétale, tel que l'Avocadofurane® (cf. la demande internationale WO 01/21150), et des agonistes de PPARs (rosiglitazone, pioglitazone).

Les composés antifongiques pouvant être utilisés dans le cadre de la présente invention en association avec l'arabinogalactane sont avantageusement l'econazole et le ketoconazole.

Les conservateurs antiseptiques pouvant être utilisés dans le cadre de la présente invention en association avec l'arabinogalactane sont par exemple le triclosan, la chlorhéxidine, les ammoniums quaternaires.

Les composés contenant des insaponifiables d'huiles végétales pouvant être utilisés dans le cadre de la présente invention en association avec l'arabinogalactane sont avantageusement choisis dans le groupe constitué par les lipides furaniques d'avocat, les insaponifiables d'avocat, les insaponifiables de soja, les insaponifiables d'avocat et de soja, les concentrats d'huile de lupin, les concentrats d'huile de tournesol et leurs mélanges.

Les lipides furaniques d'avocat pouvant être utilisés dans le cadre de la présente invention en association avec l'arabinogalactane sont avantageusement des 2-alkyl furanes naturels, notamment l'actif Avocadofurane® commercialisé par les Laboratoires Expanscience, pouvant être obtenus par le procédé décrit dans la demande internationale WO 01/21605.

Les insaponifiables d'avocat et de soja pouvant être utilisés dans le cadre de la présente invention en association avec l'arabinogalactane sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience.

Les concentrats d'huile de lupin pouvant être utilisés dans le cadre de la présente invention en association avec l'arabinogalactane sont avantageusement des concentrats obtenus par distillation moléculaire d'huile de lupin, avantageusement d'huile de lupin blanc doux, tels que ceux décrits dans la demande internationale WO 98/47479. Ils contiennent avantageusement environ 60% en poids d'insaponifiables.

Les concentrats d'huile de tournesol pouvant être utilisés dans le cadre de la présente invention en association avec l'arabinogalactane sont avantageusement des concentrats de tournesol linolé linoléiques, tels que l'actif commercialisé par les Laboratoires Expanscience, Soline® (cf. la demande internationale WO 01/21150).

Selon un aspect préféré de l'invention, la composition comprend à la fois de l'arabinogalactane et du lupéol. Selon un autre aspect préféré, la composition contient de l'arabinogalactane, du lupéol et des peptides de soja. Enfin, la composition préférée est constituée d'arabinogalactane, de lupéol, de peptides de soja et d'un extrait peptidique d'avocat. L'association de ces quatre composants confère à la composition une action anti-vergetures remarquable, ces quatre composés agissant en synergie pour protéger la peau. Une telle composition permet d'obtenir un effet particulièrement bénéfique sur les vergetures existantes, ainsi que sur la prévention de leur apparition.

La composition topique selon l'invention comprend également un véhicule approprié qui peut être tout véhicule parmi ceux connus de l'homme du métier en vue d'obtenir une composition utilisable selon l'invention, en particulier sous forme d'une crème, d'une lotion, d'un gel, d'un spray, d'un patch, d'une eau, d'une pommade, de lait ou d'huile, éventuellement sous la forme d'une émulsion, avec en outre des composants connus de l'homme du métier pour améliorer, modifier ou stabiliser la composition d'un point de vue cosmétique ou dermatologique.

Pour l'ingestion par voie orale, de nombreuses formes de réalisation et notamment des compléments alimentaires sont possibles. Leur formulation est réalisée par les procédés usuels pour produire des comprimés, des gélules, des capsules molles, des dragées, des émulsions, des gels. En particulier, l'arabinogalactane et les autres actifs de l'invention peuvent être incorporés dans toutes formes de compléments alimentaires ou d'aliments enrichis, par exemple, des barres alimentaires, des poudres compactées ou non, des boissons, des produits laitiers et en particulier des yaourts et des yaourts à boire. Les poudres peuvent être diluées dans de l'eau, des sodas, des jus de fruits, des produits laitiers ou à base de soja, de riz, ou être incorporées dans des barres alimentaires.

Les conditions opératoires pour préparer ces compositions selon l'invention font partie des connaissances générales de l'homme du métier.

Cette composition selon l'invention est particulièrement destinée pour la prévention de l'apparition des vergetures pendant la grossesse, ou d'une manière générale lorsque l'environnement hormonal est défavorable au métabolisme des fibroblastes.

Enfin, la présente invention a pour objet l'utilisation cosmétique d'arabinogalactane, ou d'une composition telle que définie ci-dessus, pour prévenir et/ou traiter l'apparition de vergetures sur la peau, en particulier au cours de la grossesse.

La présente demande décrit également l'utilisation d'arabinogalactane, ou d'une composition telle que définie ci-dessus, pour l'obtention d'une composition dermatologique destinée à prévenir et/ou traiter l'apparition de vergetures sur la peau.

Les exemples suivants sont destinés à illustrer la présente invention et ne doivent en aucun cas être interprétés comme pouvant en restreindre la portée.

## DESCRIPTION DES FIGURES

Figure 1 : Etude de la prolifération des fibroblastes en présence d'arabinogalactane (moy $DO_{570}$)

Figure 2 : Expression génique du collagène I en présence d'arabinogalactane (Quantité relative ColI)

Figure 3 : Expression génique du collagène III en présence d'arabinogalactane (Quantité relative ColIII)

Figure 4 : Effet préventif de l'arabinogalactane sur l'expression génique du collagène I dans des fibroblastes traités à l'hydrocortisone (Quantité relative ColI)

Figure 5 : Effet anti-inflammatoire de l'arabinogalactane vis à vis du PMA : dosage de l'IL1$\beta$ (DO ELISA/$DO_{540}$)

Figure 6 : Evaluation clinique de l'évolution de paramètres de couleur, d'aspect, de largeur,...des vergetures après 3 mois de traitement

Figure 7 : Evolution des vergetures en mesures centimétriques

Figure 8 : Evaluation de la mesure de degré d'hydratation

Figure 9 : Evolution du nombre de vergetures

Figure 10 : Evolution de l'érythème global

Figure 11 : Evolution de l'indice de gravité

## EXEMPLES

Les effets de l'arabinogalactane, utilisé à une concentration de 0,2% de matières sèches, a été analysée sur les paramètres suivants:

✔ La prolifération des fibroblastes ;

✔ L'expression des macromolécules dermique : Collagène I, collagène III, dans des conditions normales et dans un environnement hormonal délétère mimant celui de la grossesse ;

✔ L'inflammation.

## A. Pré-requis de tolérance cutanée

• Inflammation :

Le potentiel pro-inflammatoire de l'arabinogalactane a été évalué sur kératinocytes humains normaux. Les interleukines 1 et 8 ainsi que le TNF$\alpha$, médiateurs précoces de l'inflammation, ont été dosés dans le surnageant des cellules.

A la concentration de 0,2% Matière sèche (MS), l'arabinogalactane n'entraîne pas de relargage d'interleukines 1 et 8 ainsi que du TNF-$\alpha$. L'arabinogalactane n'est donc pas pro-inflammatoire.

## B. Méthodes d'étude de l'activité biologique

1. Etude de la prolifération des fibroblastes par la méthode au MTT

Le test au MTT [bromure de 3-(4,5-dimethylthiazol-2-yl)-2,5-diphenyltetrazolium] est un test colorimétrique qui mesure la viabilité cellulaire. Le MTT est un sel de tétrazolium hydrosoluble de couleur jaune ; les cellules métaboliquement actives sont capables de le réduire en cristaux de formazan bleus.

A J0, les fibroblastes sont ensemencés en milieu RPMI à 1% d'SVF.

A J1, les cellules sont traitées par le RPMI à 10% SVF (contrôle positif) ou par l'arabinogalactane à 0,2% pendant 48 heures.

En fin de traitement, la viabilité cellulaire est quantifiée par un test au MTT : après 3 heures de contact avec le MTT, les cristaux de formazan formés sont solubilisés par du DMSO et la densité optique, proportionnelle à la quantité de cellules métaboliquement actives donc vivantes, est lue à 570 nm contre le blanc (sans cellules).

2- Etude de l'expression des gènes codant pour les collagènes I et III par RT-PCR Principe de la PCR Quantitative en temps réel :

La PCR Quantitative en temps réel ou QRT-PCR (pour Quantitative Real Time Polymerase Chain Reaction) est une méthode qui permet de mesurer de façon spécifique et quantitative l'expression de gènes d'intérêt par amplification. La quantification est basée sur le suivi de l'amplification des gènes en temps réel en utilisant comme système rapporteur la technologie SYBR Green : molécule aux propriétés fluorescentes qui s'intercale au sein de l'ADN double brin. La PCR se déroule en une succession de cycles de température selon 3 étapes :

- Dénaturation : séparation des 2 brins d'ADN.
- Hybridation : reconnaissance d'une séquence d'ADN correspondant à un gène cible grâce aux amorces spécifiques.
- Extension : de la séquence d'intérêt par action d'une polymérase.

A la fin de la réaction, la quantification est réalisée en analysant le « cycle seuil » (Ct = point où le signal d'émission de fluorescence sera statistiquement et significativement plus élevé que le bruit de fond). Les quantités d'ADN sont comparées dans la partie exponentielle, moment pendant lequel l'augmentation de la quantité d'ADN est proportionnelle à la quantité initiale de matrice.

Protocoles

• Effet de l'arabinogalactane sur l'expression génique des collagènes I et III par des fibroblastes normaux

Les fibroblastes ont été ensemencés dans du milieu RPMI à 1%SVF. 24 heures après, les cellules ont été traitées par le TGF$\beta$1 à 5 ng/ml ou l'arabinogalactane à 0,2% pendant 48 heures.

• Effet préventif de l'arabinogalactane sur l'expression génique du collagène I dans des fibroblastes traités à l'hydrocortisone (environnement hormonal)

Les fibroblastes ont été pré-traités avec l'arabinogalactane à 0,2%. Après 24 heures d'incubation à 37°C, le traitement avec l'arabinogalactane a été renouvelé en présence de l'hydrocortisone à 50 $\mu$M pendant 48 heures.

L'hydrocortisone à 50 $\mu$M et le TGF$\beta$ à 5ng/ml ont été utilisés en tant que contrôles validant le test.

A la fin du traitement des cellules, les ARN totaux ont été extraits (kit d'extraction RNeasy MiniKit) puis dosés de façon quantitative en minichips à l'aide du système Experion (kit Experion RNA StdSens).

Les ARN totaux ont ensuite été rétro-transcrits en cDNA (kit iScript cDNA Synthesis). Enfin, les cDNA néo-synthétisés relatifs au gène d'intérêt ou aux gènes de référence (HPRT, GAPDH, YWHAZ, BETA ACTIN = normalisateurs) ont été amplifiés sélectivement par PCR en temps réel (iQ5,Biorad) en utilisant des amorces spécifiques des séquences cibles.

L'expression des gènes de référence est analysée dans les mêmes échantillons que ceux pour lesquels l'expression des gènes d'intérêt est évaluée afin de normaliser les résultats.

Analyse de résultats

Les résultats sont normalisés par rapport au gène de référence le plus stable (d'après l'algorithme geNorm):

$$\Delta Ct = Ct \text{ gène d'intérêt } - Ct \text{ gène de référence le plus stable}$$

La variation du nombre de copies du gène d'intérêt lors du traitement est ensuite calculée selon la formule suivante :

$$\Delta\Delta Ct = \Delta Ct \text{ contrôle } - \Delta Ct \text{ traitement}$$

Enfin, la quantité relative ou le niveau d'expression des gènes d'intérêt normalisé par le niveau d'expression des gènes de références dans les échantillons non traités et traités est obtenue par la formule : **QR= $2^{\Delta\Delta Ct}$**

3- Etude du potentiel anti-inflammatoire

Les kératinocytes ont été ensemencés dans du milieu KGM2. Après 24 heures d'incubation, les cellules ont été pré-traitées par l'arabinogalactane à 0,2% MS pendant 24 heures. Puis, les cellules ont été stimulées par ajout de PMA (Phorbol 12-myristate 13-acetate ; Sigma) à 10 $\mu$g/ml pendant 16 heures, afin de mimer une inflammation.

A la fin du traitement, les surnageants cellulaires ont été récoltés et l'IL1$\beta$ a été dosé à l'aide de kits ELISA (R&D Systems).

Sur les tapis cellulaires, un test au rouge neutre a été réalisé : la Densité Optique, proportionnelle à la quantité de cellules vivantes, est lue à 540 nm.

La quantité d'IL1$\beta$ est exprimée par cellules vivantes selon la formule :DO ELISA IL1$\beta$ / DO$_{540}$.

4- Statistiques

La significativité des résultats a été évaluée par un test T de Student : *p<0,05,**p<0,01.

**C. Résultats**

**Exemple 1. Effet de l'arabinogalactane sur la prolifération des fibroblastes**

La prévention et le ralentissement de l'apparition des vergetures nécessitent l'utilisation d'un actif qui favorise le renouvellement cellulaire. De plus, l'activation de la prolifération cellulaire stimule le métabolisme dermique et favorise la néosynthèse des macromolécules essentielles de la matrice extracellulaire comme les collagènes et l'élastine.

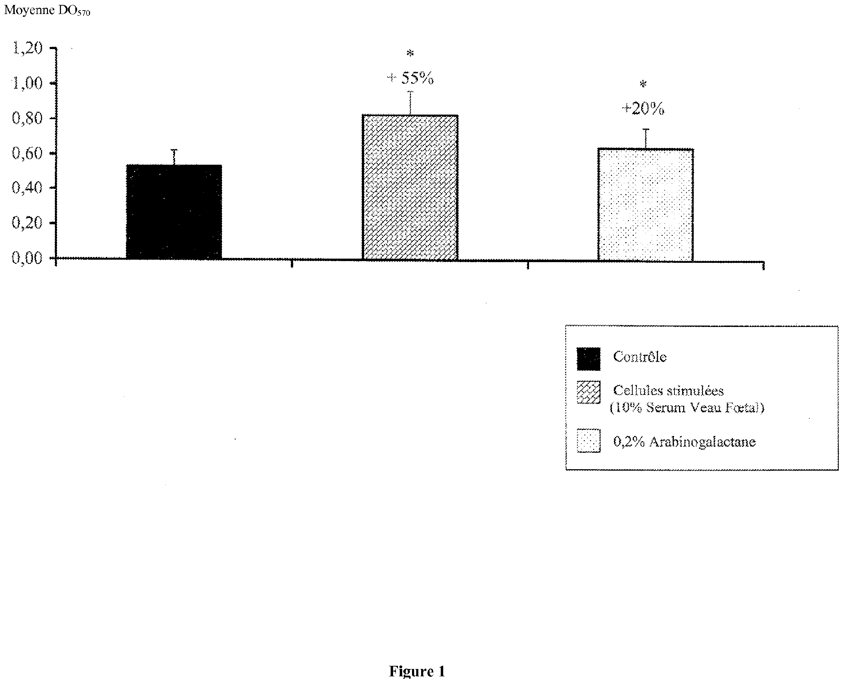

L'effet de l'arabinogalactane sur la prolifération des fibroblastes a été évalué par le test 'MTT'(figure 1). Les résultats obtenus démontrent que l'arabinogalactane active significativement la régénération cellulaire après un traitement de 48 heures (+20%). L'arabinogalactane, en favorisant ainsi la prolifération des fibroblastes, permet à ces cellules réparatrices du derme de relancer le métabolisme cellulaire, la synthèse de collagène et d'élastine dans les zones lésées.

**Exemple 2. Effet de l'arabinogalactane sur la matrice dermique**

La vergeture résulte d'une agression de la cellule fibroblastique, avec un blocage de la synthèse des macromolécules et une diminution du taux des ARN messager codant pour les collagènes I, III et pour la fibronectine. Ces altérations sont conséquentes au stress hormonal (corticoïdes) qui accompagne la grossesse. De ce fait, une atrophie générale de la matrice extracellulaire (MEC) apparaît avec un déclin du nombre de fibroblastes et une diminution des collagènes I et III.

L'effet de l'arabinogalactane a été évalué sur l'expression de deux macromolécules de la matrice dermique : collagènes I et III dans les conditions normales et dans des conditions mimant l'environnement hormonal prédisposant à l'apparition des vergetures lors de la grossesse.

**a. Effet de l'arabinogalactane sur l'expression du collagène de type I**

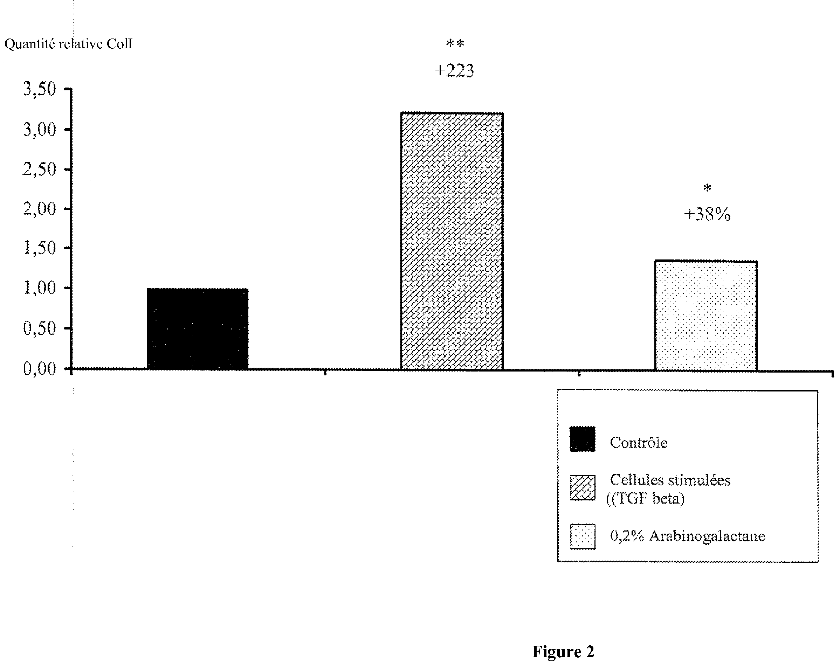

L'effet de l'arabinogalactane sur l'expression du gène codant pour le collagène de type I a été évalué par PCR en temps réel après un traitement de 48 heures. La quantité relative de collagène mesurée est normalisée par rapport à l'expression de GAPDH. Les résultats présentés dans la figure 2 montrent une augmentation significative (+38% ; $p < 0.05$) du gène codant pour le Collagène de type I en présence de l'arabinogalactane.

Le $TGF\beta$ est un facteur de croissance à fort potentiel inducteur de molécules de la matrice extracellulaire tels que les collagènes de type I et III. Il est à noter également, que dans les conditions d'essai, le $TGF\beta$ (utilisé en tant que contrôle positif) induit une très forte surexpression du gène collagène I (+223%).

**b. Effet de l'arabinogalactane sur l'expression du collagène III**

De même, après 48 heures de traitement, le $TGF\beta 1$ stimule très significativement l'expression génique du collagène de type III dans les fibroblastes (+147% d'augmentation).

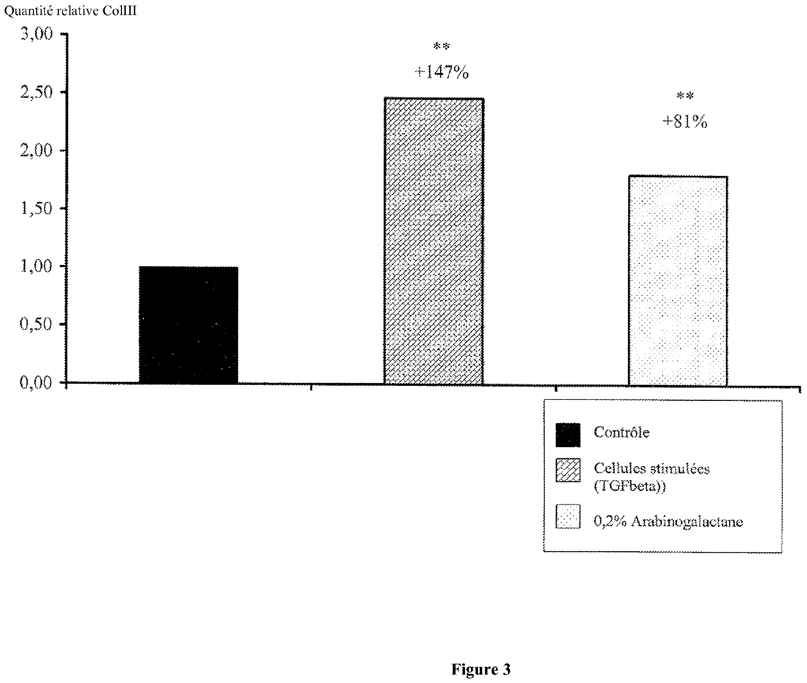

L'arabinogalactane à 0,2% MS stimule très significativement (+81%) l'expression génique du collagène de type III dans les fibroblastes (figure 3).

**c. Effet de l'arabinogalactane sur l'expression du collagène de type I dans les conditions mimant l'environnement hormonal prédisposant à l'apparition des vergetures lors de la grossesse**

L'éventuel effet préventif de l'arabinogalactane sur l'expression génique du collagène de type I a été évalué dans les conditions mimant l'environnement hormonal prédisposant aux vergetures lors de la grossesse.

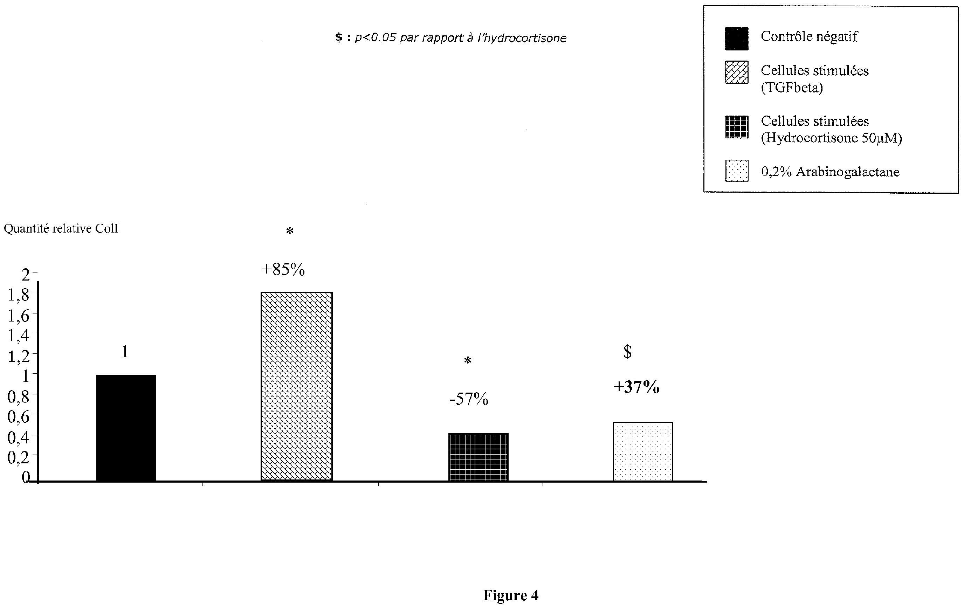

Dans la figure 4, il est confirmé que l'expression génique du collagène I diminue en présence de l'hydrocortisone (57% d'inhibition après 48 heures de traitement). En revanche, le pré-traitement des cellules pendant 24 heures par l'arabinogalactane permet de contrer l'effet inhibiteur de l'hydrocortisone et augmente l'expression du collagène I de +37%.

L'arabinogalactane stimule l'expression des collagènes de type I et III dans les conditions normales et dans les conditions favorisant l'apparition des vergetures (environnement hormonal délétère). L'arabinogalactane a donc un effet bénéfique sur les composants de la matrice extracellulaire dermique qui peuvent être altérés au cours de la grossesse et conduire à l'apparition des vergetures. Ainsi, l'arabinogalactane pourrait freiner l'apparition des vergetures.

**Exemple 3. Effet anti-inflammatoire**

La formation des vergetures s'accompagne d'une phase inflammatoire entraînant une production accrue de protéases et de cytokines inflammatoires (dont l'Interleukine 1 ou IL1). Ces enzymes et ces cytokines inflammatoires dégradent de façon importante les fibres de collagène et d'élastine de bonnes qualités et altèrent ainsi la matrice dermique. L'effet potentiellement anti-inflammatoire de l'arabinogalactane sur le relarguage de l'$IL1\beta$ a été étudié.

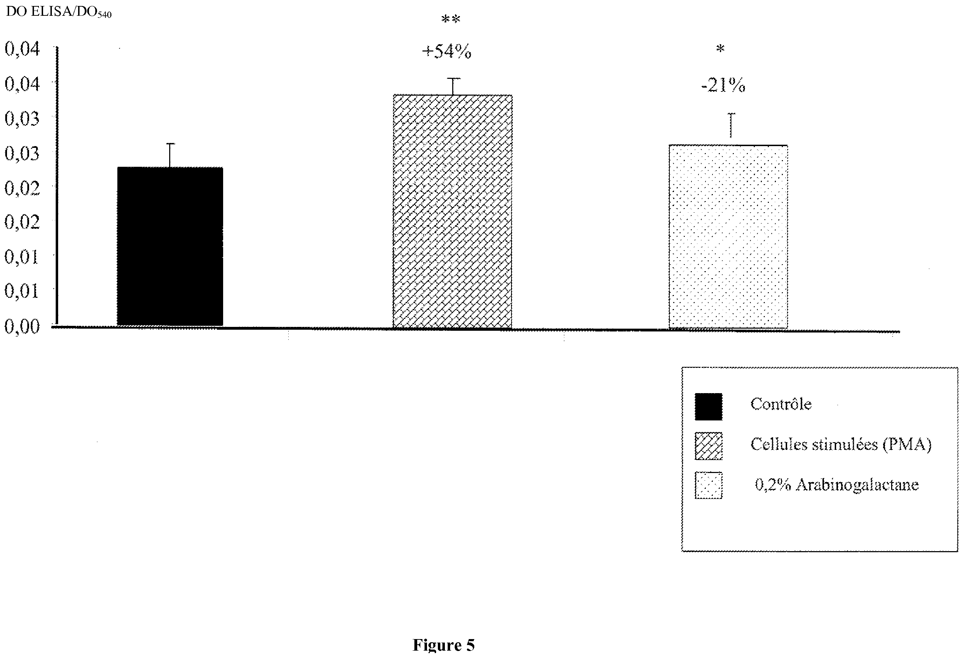

Comme le montre la figure 5, sous l'effet d'un stress au PMA, agent chimique pro inflammatoire, les kératinocytes relarguent massivement de l'$IL1\beta$, marqueur précoce de l'inflammation.

Un pré-traitement des cellules pendant 24 heures par l'arabinogalactane permet d'inhiber significativement le relargage d'$IL1\beta$ (-21%).

L'arabinogalactane exerce une action anti-inflammatoire sur la libération de cytokines inflammatoires. Il contribue ainsi à freiner la dégradation de l'armature de soutien de la peau. Cette action anti-inflammatoire contribuerait à limiter l'évolution des vergetures.

**Exemple 4. Compositions pour application par voie topique**

Les inventeurs présentent ci-dessous plusieurs compositions pour application par voie topique, particulièrement indiquées pour la prévention des vergetures pendant la grossesse.

**Composition n°1** :

| | |
|---|---|
| - ARABINOGALACTANE : | de 0.1 à 10% |
| - Lupéol : | de 0,1 à 1% |

(suite)

| | |
|---|---|
| - Peptides de soja : | de 0.1 à 4% |
| - Extrait de fleur de Sophora 22,5% | de 1 à 10% |
| - Extrait peptidique d'avocat : | de 0.1 à 1% |
| - Gluconate de zinc : | de 0.1 à 1% |
| - Beurre de karite (butyrospermum parkii butter) : | de 1 à 5% |
| - Cire de candelilla : | de 0.1 à 5% |
| - Alcool di-malate : | de 0.1 à 15% |
| - Ethylhexyl Cocoate : | de 1 à 5% |
| - Cetearylglucoside | de 1 à 5% |
| - Dicaprylyl Carbonate: | de 1 à 10% |
| - Hydrogenated Coco-Glycerides: | de 1 à 5% |
| - Glyceryl Caprylate: | de 0.1 à 1% |
| - Solution de soude à 30%: | de 0.1 à 5% |
| - Lauroyl Lysine: | de 0.1 à 1% |
| - Capryloyl Glycine: | de 0.1 à 1% |
| - Glyceryl Stearate70% & Cetearyl Alcohol 10% & Cocoglycerides10% & Cetyl Palmitate10%: | de 0.1 à 5% |
| - Potassium Cetyl Phosphate: | de 0.1 à 2% |
| - Carbomer: | de 0.1 à 2% |
| - Parfum : | de 0.1 à 1% |
| - Gomme xanthane: | de 0.1 à 1% |
| - Eau purifiée : | QSP 100% |

## Composition n°2 :

| | |
|---|---|
| - ARABINOGALACTANE : | de 0.1 à 10% |
| - Lupeol : | de 0,1% à 1% |
| - Peptides de soja hydrolysés PHYTOKINE® | de 0.1 à 5% |
| - Alcool di-malate: | de 0.1 à 15% |
| - Extrait de fleur de Sophora | de 1 à 10% |
| - Extrait peptidique d'avocat | de 0.1 à 1% |
| - Octyl Cocoate: | de 1 à 10% |
| - EMULIUM DELTA: | de 1 à 10% |
| - Panthénol Dextrogyre : | de 1 à 5% |
| - Gluconate de zinc: | de 0.1 à 1% |
| - Gluconate de cuivre : | de 0.1 à 1% |
| - Beurre de karité (liquide): | de 0.1 à 5% |
| - Cire d'abeille blanche: | de 0.1 à 5% |
| - Alcool cetylique pur: | de 0.1 à 5% |
| - Dermosoft GMCY (glyceryl caprylate): | de 0.1 à 2% |
| - Soude 30%: | de 0.1 à 5% |
| - Capryloyl glycine: | de 0.1 à 1% |
| - Carbopol ultrez 10: | de 0.1 à 2% |
| - Parfum: | de 0.1 à 1% |
| - Gomme xanthane: | de 0.1 à 1% |
| - Eau purifiée : | Qsp 100% |

L'arabinogalactane peut également être incorporée à divers produits cosmétiques tels que des eaux nettoyantes, des émulsions huile dans eau, des huiles, laits, produits moussants et sprays, dont les compositions sont présentées ci-dessous.

**EAU NETTOYANTE 1**

| Nom commercial | | % |
|---|---|---|
| EAU PURIFIÉE B4 | | QSP 100 % |
| BIOSACCHARID GUM | | De 1 à 5 % |
| BUTYLENE GLYCOL | | De 1 à 5 % |
| SAPONINE PURIFIEE | | De 0 à 1 % |
| EAU DE ROSE | | De 0 à 1 % |
| ARABINOGALACTANE | | De 0 à 5 % |
| CONSERVATEURS | | De 0 à 1 % |
| ALLANTOINE | | De 0 à 1 % |
| ACIDE CITRIQUE MONOHYDRATE | | De 0 à 1 % |
| TROMETHAMINE | | De 0 à 1 % |

**EAU NETTOYANTE 2**

| Matière première / Nom commercial | | % |
|---|---|---|
| CAPRYLOYL GLYCINE | | De 0 à 1 % |
| LESSIVE SOUDE | | De 0 à 1 % |
| EAU PURIFIÉE B4 | | De 20 à 100 % |
| SEQUESTRANT | | De 0 à 1 % |
| BUTYLENE GLYCOL | | De 1 à 5 % |
| ARABINOGALACTANE | | De 0 à 5 % |
| OCTANEDIOL | | De 0 à 1 % |
| PEG-32 | | De 1 à 5 % |
| PEG-7 PALMCOCOATE | | De 1 à 5 % |
| GLUCONATE ZINC | | De 0 à 1 % |
| ACIDE CITRIQUE MONOHYDRATE | | De 0 à 1 % |
| EAU PURIFIÉE B4 | | QSP 100 % |
| PARFUM | | De 0 à 1 % |
| POLOXAMER 184 | | De 1 à 5 % |
| D.S.B. C SP | | De 1 à 5 % |

**EMULSION Eau dans Huile**

| Matière première / Nom commercial | | % |
|---|---|---|
| ISOPARAFFINE LIQUIDE | | De 5 à 20 % |
| STEARATE D'ISOCETYLE | | De 5 à 20 % |
| HYDROXYSTEARATE AL - MG | | De 5 à 20 % |
| ABIL WE 09 | | De 1 à 5 % |
| GLYCEROL | | De 1 à 5 % |
| HUILE VASELINE EPAISSE | | De 1 à 5 % |

(suite)

| Matière première / Nom commercial | | % |
|---|---|---|
| ZINC OXYDE MICRONISE | | De 1 à 5 % |
| BUTYLENE GLYCOL | | De 1 à 5 % |
| ARABINOGALACTANE | | De 0 à 5 % |
| ISONONYL ISONONANOAT | | De 1 à 5 % |
| CIRE D'ABEILLE BLANCHE | | De 1 à 5 % |
| TARTRATE DE SODIUM | | De 1 à 5 % |
| CHLORURE DE SODIUM | | De 0 à 5 % |
| GLYCINE | | De 1 à 5 % |
| OCTANEDIOL | | De 0 à 1 % |
| CHOLESTEROL | | De 0 à 1 % |
| PHYTOSPHINGOSINE | | De 0 à 1 % |
| ACIDE TARTRIQUE | | De 0 à 1 % |
| EAU PURIFIÉE B4 | | QSP 100 % |

**EMULSION Huile dans Eau**

| Matière première / Nom commercial | | % |
|---|---|---|
| HYDROGENATED POLYDECENE | | De 5 à 20 % |
| LAURYLGLUCOSIDE-GLYSTEARATE | | De 1 à 5 % |
| DICAPRYLYL CARBONATE | | De 1 à 5 % |
| GLYCEROL | | De 5 à 20 % |
| CARBOPOL ETD 2020 | | De 0 à 1 % |
| GOMME XANTHANE | | De 0 à 1 % |
| ARABINOGALACTANE | | De 0 à 5 % |
| LESSIVE SOUDE | | De 0 à 1 % |
| CONSERVATEURS | | De 0 à 1 % |
| ACIDE CITRIQUE MONOHYDRATE | | De 0 à 1 % |
| EAU PURIFIÉE B4 | | QSP 100 % |

**HUILE**

| Matière première / Nom commercial | | % |
|---|---|---|
| SOLUBILISANT | | De 0 à 1 % |
| HUILE AMANDE DOUCE | | De 5 à 20 % |
| CAPRYLATE / CAPRATE DE COPRAH | | QSP 100 % |
| HUILE DE MACADAMIA RAFFINEE | | De 5 à 20 % |
| CAPRYLO CAPRATE DE GLYCEROL | | De 5 à 20 % |
| AALPHA BISABOLOL NAT | | De 0 à 1 % |
| ALPHA TOCOPHEROL | | De 0 à 1 % |

(suite)

| Matière première / Nom commercial | | % |
|---|---|---|
| ARABINOGALACTANE | | De 0 à 5 % |
| CONSERVATEUR | | De 0 à 1 % |
| ESTER | | De 0 à 1 % |

**LAIT**

| Matière première / Nom commercial | | % |
|---|---|---|
| HUILE AMANDE DOUCE | | De 1 à 5 % |
| HUILE MAIS | | De 1 à 5 % |
| ACIDE STEARIQUE | | De 1 à 5 % |
| ALCOOL CETYLIQUE C16 C18 | | De 0 à 1 % |
| ANTIMOUSSE 70414 | | De 0 à 1 % |
| ALCOOL LAURIQUE 11OE | | De 1 à 5 % |
| MONOLAURATE PEG 300 | | De 0 à 1 % |
| MONOLEATE DE GLYCEROL | | De 0 à 1 % |
| MONOSTEARATE DE GLYCEROL | | De 1 à 5 % |
| ARABINOGALACTANE | | De 0 à 5 % |
| CONSERVATEURS | | De 0 à 1 % |
| ACIDE CITRIQUE MONOHYDRATE | | De 0 à 1 % |
| CITRATE TRISODIQUE | | De 0 à 1 % |
| EAU PURIFIEE | | QSP 100 % |
| PARFUM | | De 0 à 1 % |
| HUILE ARACHIDE | | De 1 à 5 % |
| HUILE PALMISTE HYDROGENEE | | De 1 à 5 % |

**MOUSSANT**

| Matière première / Nom commercial | | % |
|---|---|---|
| EAU PURIFIÉE B4 | | QSP 100 % |
| LAUROAMPHOACETATE | | De 5 à 20 % |
| COCOGLUCOSIDE | | De 5 à 20 % |
| ORONAL LCG | | De 5 à 20 % |
| HYDRIOSUL KMG 30 (2) | | De 5 à 20 % |
| DISTEARATE DE PEG 6000 | | De 1 à 5 % |
| CONSERVATEUR | | De 1 à 5 % |
| ARABINOGALACTANE | | De 0 à 5 % |
| EXTRAIT CAMOMILLE | | De 1 à 5 % |
| ACIDE CITRIQUE MONOHYDRATE | | De 0 à 1 % |
| SEQUESTRANT | | De 0 à 1 % |

(suite)

| Matière première / Nom commercial | | % |
|---|---|---|
| COCOPROTEINE BLE | | De 0 à 1 % |
| PARFUM MUSTITI 11/1 | | De 0 à 1 % |
| LESSIVE SOUDE | | De 0 à 1 % |

**SPRAY**

| Matière première / Nom commercial | | % |
|---|---|---|
| EAU PURIFIÉE B4 | | QSP 100 % |
| TRILAURETH-4 PHOSPHATE | | De 1 à 5 % |
| DICAPRYLYL CARBONATE | | De 1 à 5 % |
| BUTYLENE GLYCOL | | De 1 à 5 % |
| ERYTHRITYL ESTER | | De 1 à 5 % |
| HUILE VASELINE FLUIDE | | De 1 à 5 % |
| BEURRE DE KARITE (LIQUIDE) | | De 0 à 1 % |
| JOJOBA PURE | | De 0 à 1 % |
| CONSERVATEURS | | De 0 à 1 % |
| ARABINOGALACTANE | | De 0 à 5 % |
| LESSIVE SOUDE | | De 0 à 1 % |
| PARFUM MUSTITI 10/3 | | De 0 à 1 % |
| GOMME XANTHANE | | De 0 à 1 % |
| CARBOPOL 981 NF | | De 0 à 1 % |
| SEQUESTRANT | | De 0 à 1 % |
| ACIDE CITRIQUE MONOHYDRATE | | De 0 à 1 % |

### Exemple 5. Compositions pour administration par voie orale

L'arabinogalactane est intégré à des compositions orales, dans des compositions permettant l'administration de 50 mg à 200 mg d'arabinogalactane par jour.

### 5.1 Composition anti-vergetures sous forme de capsules molles

**A- Composition 1**

| | |
|---|---|
| - Arabinogalactane | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine | QSP 1 capsule molle |

Cette composition est administrée de 4 à 6 capsules de 500 mg par jour.

### B- Composition 2

| | |
|---|---|
| - Arabinogalactane | 30 mg |
| - Huile de céréale riche en céramides et lipides polaires | 300 mg |
| - Huile de lupin | 50 mg |
| - Vitamine E | QSP 100 % de l'AJR |
| - Vitamine C | QSP 50 % de l'AJR |
| - Cire d'abeille | |
| - Lécithine de soja | |
| - Gélatine alimentaire | |
| - Glycérine | QSP 1 capsule molle. |

Cette composition est administrée de 4 à 6 capsules de 500 mg par jour.

### 5.2. comprimés

| | |
|---|---|
| - Arabinogalactane | 25 mg |
| - Extraits de céréales (blé, sarrasin, millet, épeautre) riche en acides aminés soufrés | 200 mg |
| - Vitamine C | QSP 50 % des AJR |
| - Glycosaminoglycanes issus de cartilages de poissons | 200 mg |
| - Glucidex IT 19 (agent de compression) | QSP |
| | 1 comprimé de 800 mg |

Cette composition est administrée de 5 à 8 comprimés par jour.

| | |
|---|---|
| - Arabinogalactane | 200 mg |
| - Extraits de céréales (blé, sarrasin, millet, épeautre) riche en acides aminés soufrés | 200 mg |
| - Zn sous forme de chélate | QSP 100 % des AJR. |
| - Vitamine C | QSP 50 % des AJR |
| - Glycosaminoglycanes issus de cartilages de poissons | 200 mg |
| - Arôme fruit (agrume, fruit rouge), acésulfame de potassium, Glucidex IT 19 (agent de compression) | QSP |
| | 1 comprimé de 2000 mg |

Cette composition est administrée 1 fois par jour.

### 5.3. Exemples en stick poudre

| | |
|---|---|
| - Arabinogalactane | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - ascorbate de sodium | 0,3 mg |
| - maltodextrine | QSP 5 g. |

Cette composition est administrée 2 fois par jour.

| | |
|---|---|
| - Arabinogalactane | 100 mg |
| - Extrait de centella asiatica | 100 mg |
| - Magnesium, sélénium, manganèse | QSP 100 % des AJR. |
| - Gomme xanthane | 1 mg |
| - ascorbate de sodium | 0,3 mg |
| - maltodextrine | QSP 5 g. |

Cette composition est administrée 2 fois par jour.

**5.4. Exemple en barre céréalière goût chocolat**

| | |
|---|---|
| - Arabinogalactane | 200 mg |
| - Lycopène | 6 mg, |
| - astaxanthine | 4 mg |
| - fucoxanthine | 4 mg |
| - lutéine sous forme micro-encapsulée | 4 mg. |
| - Tocotriénol micro-encapsulés | QSP 100 % AJR en vitamine E. |
| - Chocolat noir, oligofructose, sucre, sirop de fructose, cacao maigre en poudre, céréales croustillantes, lait écrémé en poudre, amandes, glycérol, sorbitol, huiles végétales, sirop de glucose, arôme, lait condensé sucré, lécithine de soja, mono-et diglycérides d'acides gras, sirop caramélisé, maltodextrine, sel, sorbate de potassium, alpha tocophérol. | QSP une barre de 50 g |

Cette composition est administrée une fois par jour.

**5.5. Exemple en barre céréalière goût vanille**

| | |
|---|---|
| - Arabinogalactane | 200 mg |
| - Extraits de céréales (blé, sarrasin, millet, épautre) riche en acides aminés soufrés | 200 mg |
| - Glycosaminoglycanes issus de cartilages de poissons | 200 mg |
| - Extrait de thé vert riche en polyphénols | 200 mg |
| - Oligofructose, sucre, sirop de fructose, céréales croustillantes, lait écrémé en poudre, amandes, glycérol, sorbitol, huiles végétales, sirop de glucose, arôme, lait condensé sucré, lécithine de soja, mono-et diglycérides d'acides gras, sirop caramélisé, maltodextrine, sel, sorbate de potassium, alpha tocophérol. | QSP une barre de 50 g |

Cette composition est administrée une fois par jour.

**5.6. Exemples en boisson lactée goût praliné**

| | |
|---|---|
| - Arabinogalactane | 200 mg |
| - Extrait de thé vert riche en polyphénols | 100 mg. |
| - Vitamine du groupes B (B1, B2, B3, B5, B6, B9, B12) | QSP 100 % des AJR. |
| - Zn, Mg, Se | QSP 100 % des AJR. |

(suite)

| - Lait écrémé en poudre, arôme, fructose, blanc d'oeuf, noisettes, sucre, caramel, béta-carotène, gomme xanthane, aspartame, acésulfame de potassium, lécithine de soja, maltodextrine | QSP un sachet de 30 g |
|---|---|

Cette composition est administrée une fois par jour.

## Exemple 6: Evaluation d'une composition cosmétique anti-vergeture (composition n°2 de l'exemple 4 ci-dessus) dans un modèle de peau reconstruite mimant une vergeture.

Contexte et objectif de l'étude

La formation de la vergeture implique un remaniement de la matrice extracellulaire dermique avec un appauvrissement et une altération des macromolécules (comme le collagène et l'élastine) la composant associé à un processus inflammatoire. La vergeture peut alors être comparée à une cicatrice atrophique.

Le processus de cicatrisation se déroule selon différentes étapes impliquant à la fois les kératinocytes épidermiques et les fibroblastes dermiques. Ces étapes sont caractérisées par des modifications biochimiques, moléculaires et morphologiques qui peuvent être identifiées en quantifiant l'expression de bio-marqueurs spécifiques.

La Demanderesse a développé un modèle expérimental in vitro de cicatrice dermique mimant la vergeture en utilisant le modèle de peau totale reconstruite (modèle Phenion®, Duesseldorf, Germany) afin d'évaluer l'efficacité d'une composition cosmétique anti-vergeture.

Méthodologie

Trois conditions ont été réalisées :

Condition a : condition **témoin** de peau reconstruite non lésée (sans plaie dermique) et non traitée avec la composition cosmétique.

Condition b : condition de peau reconstruite lésée sur laquelle une « **plaie** » **dermique** a été réalisée, mais sur laquelle la composition cosmétique n'a pas été appliquée.

Condition c : condition **traitée avec la composition cosmétique anti-vergeture** qui a été déposée à la surface de la peau reconstruite (application topique) juste après avoir réalisé la « plaie » dermique.

L'expression génique des bio-marqueurs suivants a été mesurée 24h, 48h, 8 jours et 16 jours après lésion du tissu par PCR quantitative en temps réel utilisant la technologie TaqMan® : les Collagènes de type I et de type VII qui stimulent le processus de cicatrisation permettant un remodelage rapide de la matrice extracellulaire, l'Intégrine beta 1 qui agit sur la migration des kératinocytes, étape précoce de la réparation tissulaire et l'Elastine qui renforce le tissu cutané et qui est dégradée lors des phénomènes inflammatoires. Les trois conditions ont été réalisées en duplicata durant deux expériences indépendantes nommées EXP N°1 et EXP N°2.

Résultats et conclusion

Les données du tableau I ci-dessous représentent les Quantités Relatives de chaque gène d'intérêt (normalisées par le gène de référence GAPDH) exprimé dans la condition c (peau avec « plaie » traitée avec la composition cosmétique anti-vergeture) par rapport à la condition b (peau avec « plaie » non traitée).

Tableau I : Expression des gènes Collagène I, Collagène VII, Intégrine beta1 et Elastine dans un modèle de peau reconstruite mimant une vergeture - Effet de la composition cosmétique anti-vergeture.

| Condition testée | Bio-marqueur analysé | N° Expérience | Quantité Relative | | | |
|---|---|---|---|---|---|---|
| | | | **24h** | **48h** | **8j** | **16j** |
| b : « plaie » dermique | Tous les marqueurs | EXPs 1 et 2 | 1 | 1 | 1 | 1 |

(suite)

| Condition testée | Bio-marqueur analysé | N° Expérience | Quantité Relative | | | |
|---|---|---|---|---|---|---|
| | | | 24h | 48h | 8j | 16j |
| c : plaie traitée avec la composition cosmétique anti-vergeture | Collagène I | EXP N°2 | 0,84 | 1,26 | **1,70** | 0,75 |
| | Collagène VII | EXP N° 1 | 1,13 | 0,96 | **3,08** | **4,94** |
| | Intégrine beta 1 | EXP N° 1 | 1,26 | 0,83 | **1,72** | **2,31** |
| | | EXP N°2 | 0,72 | 2,04 | **2,29** | **3,13** |
| | Elastine | EXP N°1 | 1,61 | 0,42 | **2,24** | 0,66 |
| | | EXP N°2 | 1,86 | 2,09 | **5,2** | 0,66 |

Dans ce modèle de peau mimant une vergeture (avec « plaie » dermique), l'application topique de la composition cosmétique anti-vergeture est capable, à partir de 8 jours, d'augmenter l'expression des Collagènes I et VII, de l'Intégrine beta 1 et l'Elastine (de X1,7; X3,08; X2,29; X5,2 respectivement). Cet effet est amplifié jusqu'au 16ème jour pour le Collagène VII et l'Intégrine beta 1 (X4,9 et X3 respectivement).

Ainsi la composition cosmétique anti-vergeture testée montre, dans ce modèle mimant la vergeture, un double mécanisme d'action basé sur :

- La régénération tissulaire, en stimulant les communications intercellulaires au niveau de la jonction dermo-épidermique, permettant une ré-organisation précose du tissu cutané et contrant la dégradation matricielle (Intégrine beta1 et Collagène VII);
- Le remodellage de la matrice dermique, en redonnant une certaine résistance et force à la matrice extracellulaire du derme (Collagène I et Elastine).

**Exemple 7: Evaluation de l'effet d'un traitement topique de vergetures récentes chez des femmes après accouchement**

La Demanderesse a étudié la réponse de vergetures récentes, cliniquement actives, à une application topique d'une crème formulée de manière spécifique pour réduire les vergetures.

Le crème correspond à la composition n° 2 de l'exemple 4 ci-dessus.

**Protocole**

Conception de l'étude

Dans une étude comparative en simple-aveugle, randomisée, intra-individuelle, 22 femmes post-partum présentant des vergetures (marques d'étirement) récentes symétriques et comparables (Stade I dans la classification de Deprez-Adatto : cf. Tableau 2 ci-dessous) sur chacune de leurs cuisses ou hanches au niveau de la ligne de base, ont appliqué la crème avec des massages circulaires doux deux fois par jour sur une de leurs cuisses pendant 3 mois. L'autre cuisse a servi de témoin non traité. Chaque cuisse a été évaluée mensuellement par des examens cliniques et par une mesure instrumentale. Les femmes ont également appliqué la crème sur toutes les zones concernées par des vergetures pour obtenir une évaluation globale de l'efficacité.

Les femmes ayant participé à l'étude avaient un indice de masse corporelle IMC normal (19 et 25 kg.m$^{-2}$) et avaient entre 18 et 40 ans.

Le niveau de signification a été fixé à 5 % (test de Student et test de Wilcoxon).

Tableau 2: Classification de vergetures (Deprez-Adatto)

| Cf. Adatto M.A., Deprez P., Journal of Cosmetic Dermatology, 2 (2) : 61 - 67, avril 2003. | |
|---|---|
| **Classification clinique des vergetures** | **Aspect Clinique** |
| Stade I | Vergetures récentes, inflammatoires, habituellement violacées. |

(suite)

| Cf. Adatto M.A., Deprez P., Journal of Cosmetic Dermatology, 2 (2) : 61 - 67, avril 2003. | |
|---|---|
| **Classification clinique des vergetures** | **Aspect Clinique** |
| Stade II a | Vergetures blanches, superficielles sans stries transversales et sans dépression palpable à la surface de la peau. |
| Stade II b | Vergetures blanches, superficielles sans stries transversales mais avec dépression palpable à la surface de la peau. |
| Stade III a | Vergetures blanches à fond atrophique avec stries transversales mesurant moins de 1 cm de large, sans aspect nacré en profondeur. |
| Stade III b | Vergetures blanches à fond atrophique avec stries transversales mesurant moins de 1 cm de large, avec aspect nacré en profondeur. |
| Stade IV | Vergetures blanches à fond atrophique avec stries transversales mesurant plus de 1 cm de large, avec aspect nacré en profondeur. |

Critères d'évaluation

Les critères d'évaluation sont présentés dans le tableau 3 ci-dessous

Tableau 3

| Évaluation | Analyse | J0 | J28 | J56 | J84 |
|---|---|---|---|---|---|
| Évaluation clinique à partir d'échelles prédéfinies | traité/non traité | X | X | X | X |
| Mesures centimétriques des vergetures | traité/non traité | X | X | X | X |
| Mesure de degré d'hydratation avec un Corneometer® | traité/non traité | X | X | X | X |
| Évaluation subjective par questionnaire | avant/après | X | X | X | X |
| Macrophotographies en lumière parallèle | avant/après | X | | | X |
| Photographies polarisées croisées (Nikon D70) = évaluation de la composante érythémateuse | avant/après | X | | | X |
| Score de tolérance globale | avant/après | X | | | X |
| Recueil d'événement indésirable | avant/après | X | X | X | X |

**Résultats** :

Efficacité de la crème sur les vergetures post-partum récentes

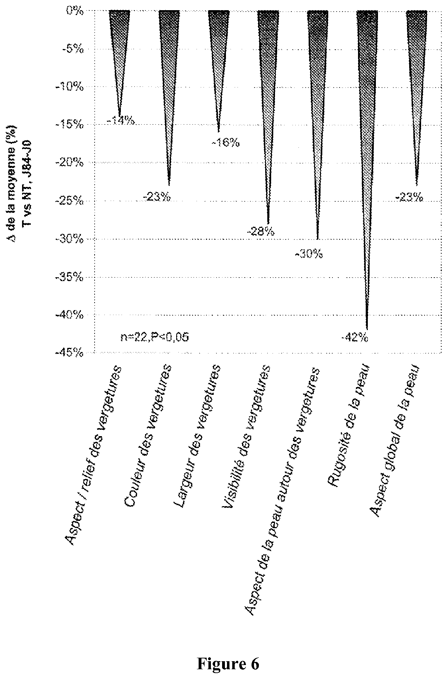

La figure 6 correspond à l'évaluation clinique de l'évolution de paramètres de couleur, d'aspect, de largeur, ... des vergetures après 3 mois de traitement.

Après une période d'application de 3 mois de la composition, on a observé que tous les paramètres évalués (Aspect/relief des vergetures, couleur des vergetures, largeur des vergetures, visibilité des vergetures, aspect de la peau autour des vergetures, rugosité de la peau autour des vergetures, aspect global de la peau) sont statistiquement améliorés. En outre, la peau a semblé plus ferme (+31 %) et est apparue plus hydratée (+50 %). Globalement, le clinicien a observé une amélioration globale significative pour 100 % des sujets : 31 % ont présenté une amélioration importante et 69 % une amélioration moyenne.

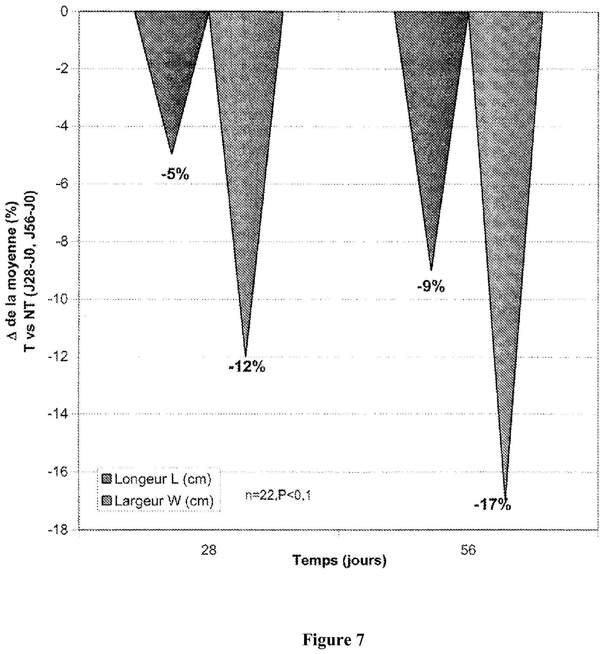

La figure 7 correspond à l'évolution des vergetures en mesures centimétriques.

Sur cette figure, on observe une diminution significative des dimensions des vergetures traitées (longueur et largeur).

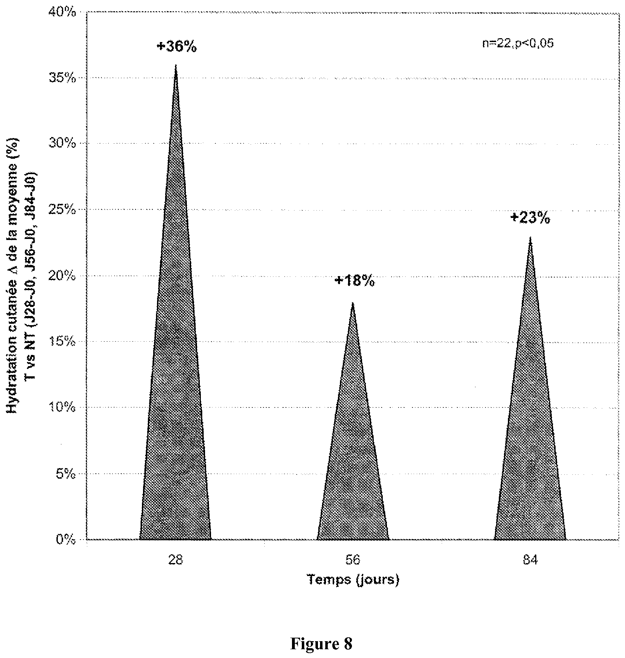

La figure 8 correspond à l'évaluation de la mesure de degré d'hydratation.

Sur cette figure, les mesures ont démontré une amélioration significative (+23 %) du niveau d'hydratation de la peau après un test de 3 mois.

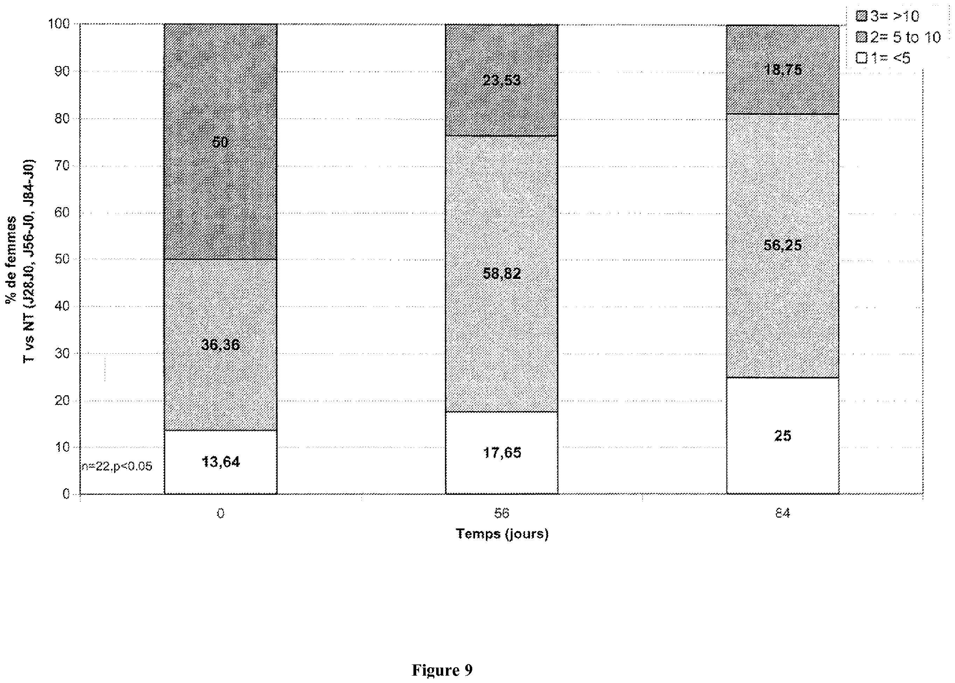

Les figures 9, 10 et 11 correspondent à l'évolution de la sévérité des vergetures.

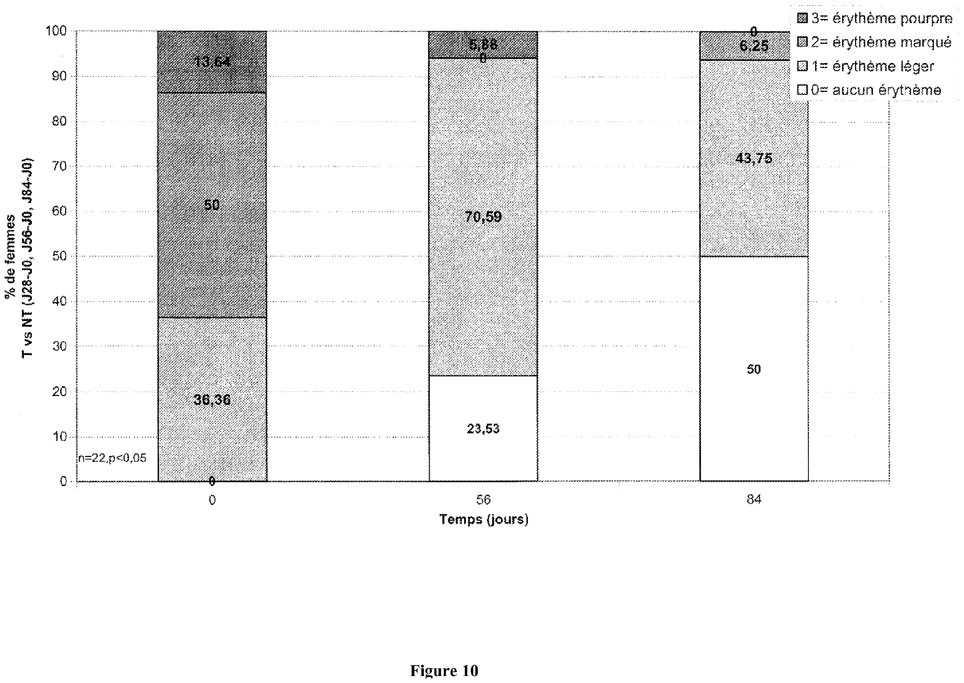

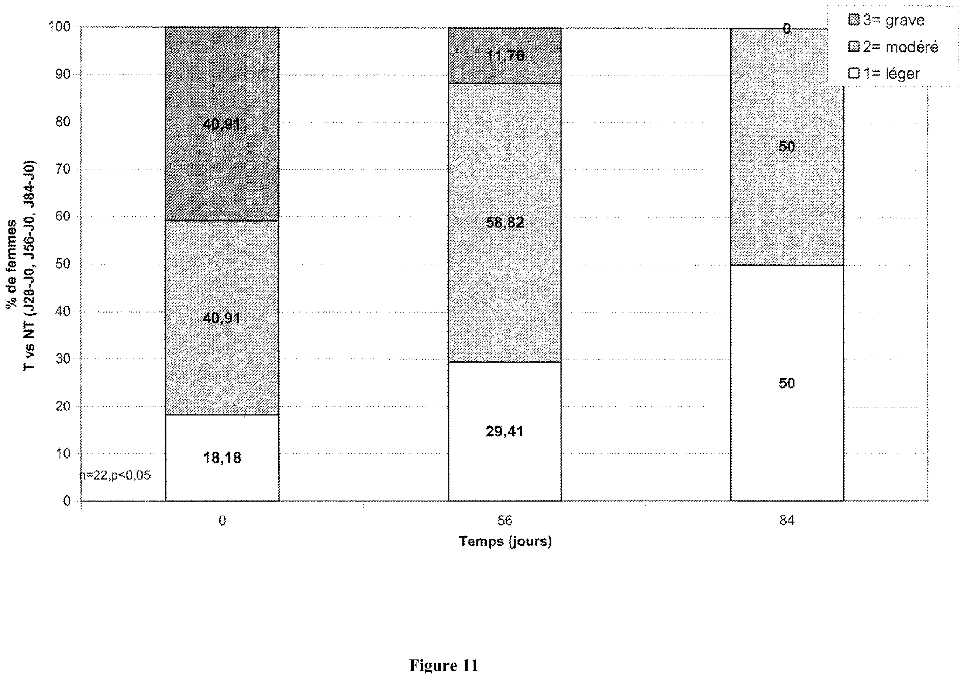

Plus précisément, la figure 9 montre l'évolution du nombre de vergetures, la figure 10 montre l'évolution de l'érythème global, et la figure 11 montre l'évolution de l'indice de sévérité.

Après 3 mois, le clinicien a observé une amélioration réelle sur les vergetures : une diminution significative de l'érythème (observée chez 75 % des femmes) et de l'indice de gravité (observées chez 87 % des femmes).

Le tableau 4 ci-dessous présente les résultats d'auto-évaluation et indique le pourcentage d'accord (totalement d'accord et d'accord).

Tableau 4

| Efficacité sur les vergetures | Après 3 mois |
|---|---|
| Une diminution du relief des vergetures | 81 % |
| Une amélioration d'aspect de la peau autour des vergetures | 81 % |
| Une amélioration de la couleur des vergetures | 100 % |
| Une amélioration de l'aspect global des vergetures | 88 % |
| Une amélioration de l'aspect global de la peau | 88 % |
| Une peau plus souple | 81 % |
| Une peau plus lisse | 75 % |
| Une peau plus douce | 88 % |
| Vergetures moins visibles | 75 % |
| Le produit a une efficacité sur les vergetures | 94 % |

Les réponses données au questionnaire d'évaluation subjectif ont nettement confirmé les résultats précédents. Le produit a été apprécié pour son efficacité sur les vergetures, la qualité de la peau, et également pour ses propriétés organoleptiques.

Taux de satisfaction globale : **88 %** des volontaires souhaiteraient poursuivre l'utilisation de la crème.

Le produit présente une très bonne tolérance cutanée. Aucun évènement indésirable n'a été rapporté au cours de l'étude.

**Conclusion**

Cette composition anti-vergetures, contenant de l'Arabinogalactane, ainsi que du Lupéol et des biopeptides naturels, a un effet statistiquement significatif sur des vergetures récentes actives chez des femmes post-partum. En outre, on observe une amélioration dès 28 jours qui se poursuit jusqu'à 3 mois.

La composition procure ainsi :

➢ Une nette amélioration de la qualité globale de la peau aussi bien que des vergetures.
➢ Une amélioration globale significative de la sévérité des vergetures.
➢ Une diminution significative des dimensions des vergetures traitées (largeur et longueur).
➢ Une diminution visible de l'érythème global.
➢ Un bon profil de tolérance et de sécurité.

**Revendications**

1. Utilisation cosmétique d'arabinogalactane pour prévenir et/ou traiter l'apparition de vergetures sur la peau, en particulier au cours de la grossesse.

2. Utilisation selon la revendication 1, **caractérisée en ce que** l'arabinogalactane est dans une composition qui est administrée par voie topique.

3. Utilisation selon la revendication 1, **caractérisée en ce que** l'arabinogalactane est dans une composition qui est administrée par voie orale.

**4.** Utilisation selon l'une des revendications 1 à 3, **caractérisée en ce que** l'arabinogalactane est extrait de mélèze.

**5.** Utilisation selon l'une des revendications 1 à 4, **caractérisée en ce que** l'arabinogalactane est présent dans une composition dans une proportion comprise entre 0,01% et 10% en poids par rapport au poids total de la composition.

**6.** Utilisation selon l'une des revendications 2, 3 ou 5, **caractérisée en ce que** ladite composition comprend au moins un autre agent anti-vergetures choisi parmi le lupéol, les peptides de soja, les tripeptides constitués des acides aminés Glycine, Histidine et Lysine, l'extrait de fleur de sophora, l'extrait de chlorophycée, l'extrait peptidique d'avocat, le panthénol, et les mélanges de ces composés.

**7.** Utilisation selon l'une des revendications 2, 3, 5 ou 6 **caractérisée en ce que** ladite composition est destinée à la prévention de l'apparition des vergetures pendant la grossesse.

**8.** Utilisation selon l'une des revendications 1 à 7, **caractérisée en ce que** l'arabinogalactane est dans une composition comprenant en outre du lupéol, des peptides de soja et un extrait peptidique d'avocat.

**9.** Composition cosmétique comprenant de l'arabinogalactane, du lupéol, des peptides de soja et un extrait peptidique d'avocat.

**Patentansprüche**

**1.** Kosmetische Verwendung von Arabinogalactan zur Vorbeugung und/oder Behandlung des Auftretens von Dehnungsstreifen auf der Haut, insbesondere während der Schwangerschaft.

**2.** Verwendung nach Anspruch 1, **dadurch gekennzeichnet, dass** das Arabinogalactan in einer Zusammensetzung ist, die auf topischem Weg verabreicht wird.

**3.** Verwendung nach Anspruch 1, **dadurch gekennzeichnet, dass** das Arabinogalactan in einer Zusammensetzung ist, die auf oralem Weg verabreicht wird.

**4.** Verwendung nach einem der Ansprüche 1 bis 3, **dadurch gekennzeichnet, dass** das Arabinogalactan Lärchenextrakt ist.

**5.** Verwendung nach einem der Ansprüche 1 bis 4, **dadurch gekennzeichnet, dass** das Arabinogalactan in einer Zusammensetzung in einem Anteil zwischen 0,01 % und 10 Gew.-% inklusive im Verhältnis zum Gesamtgewicht der Zusammensetzung vorhanden ist.

**6.** Verwendung nach einem der Ansprüche 2, 3 oder 5, **dadurch gekennzeichnet, dass** die Zusammensetzung mindestens ein anderes Anti-Dehnungsstreifen-Mittel umfasst, ausgewählt aus dem Lupeol, den Sojapeptiden, den Tripeptiden, gebildet aus den Amionsäuren Glycin, Histidin und Lysin, dem Sophorablütenextrakt, dem Chlorophyceaeextrakt, dem Peptidextrakt von Avocado, dem Panthenol und den Gemischen dieser Verbindungen.

**7.** Verwendung nach einem der Ansprüche 2, 3, 5 oder 6, **dadurch gekennzeichnet, dass** die Zusammensetzung zur Vorbeugung des Auftretens von Dehnungsstreifen während der Schwangerschaft bestimmt ist.

**8.** Verwendung nach einem der Ansprüche 1 bis 7, **dadurch gekennzeichnet, dass** das Arabinogalactan in einer Zusammensetzung ist, die ferner Lupeol, Sojapeptide und einen Peptidextrakt von Avocado umfasst.

**9.** Kosmetische Zusammensetzung, umfassend Arabinogalactan, Lupeol, Sojapeptide und einen Peptidextrakt von Avocado.

**Claims**

**1.** Cosmetic use of arabinogalactan to prevent and/or treat the appearance of stretch marks on the skin, in particular during pregnancy.

**2.** Use according to claim 1, **characterized in that** the arabinogalactan is in a composition which is administered topically.

**3.** Use according to claim 1, **characterized in that** the arabinogalactan is in a composition which is administered orally.

**4.** Use according to one of claims 1 to 3, **characterized in that** the arabinogalactan is extracted from larch.

**5.** Use according to one of claims 1 to 4, **characterized in that** the arabinogalactan is present in a composition in a proportion between 0.01% and 10% by weight compared to the total weight of the composition.

**6.** Use according to one of claims 2, 3 or 5, **characterized in that** the aforesaid composition contains at least one other anti-stretch mark agent selected from lupeol, soya peptides, tripeptides composed of the amino acids Glycine, Histidine and Lysine, sophora flower extract, chlorophyceae extract, peptide extract of avocado, panthenol, and mixtures of said compounds.

**7.** Use according to one of claims 2, 3, 5 or 6 **characterized in that** the aforesaid composition is intended to prevent the appearance of stretch marks during pregnancy.

**8.** Use according to one of claims 1 to 7, **characterized in that** the arabinogalactan is in a composition further containing lupeol, soya peptides and a peptide extract of avocado.

**9.** Cosmetic composition containing arabinogalactan, lupeol, soya peptides and a peptide extract of avocado.

Moyenne $DO_{570}$

Légende:
- ■ Contrôle
- ▨ Cellules stimulées (10% Serum Veau Fœtal)
- ▦ 0,2% Arabinogalactane

**Figure 1**

Quantité relative ColI

**

+223

*

+38%

Légende:
- Contrôle
- Cellules stimulées ((TGF beta)
- 0,2% Arabinogalactane

**Figure 2**

EP 2 346 486 B1

Quantité relative ColIII

Figure 3

$ : p<0.05 par rapport à l'hydrocortisone

Contrôle négatif

Cellules stimulées
(TGFbeta)

Cellules stimulées
(Hydrocortisone 50µM)

0,2% Arabinogalactane

Quantité relative ColI

**Figure 4**

DO ELISA/DO$_{540}$

**Figure 5**

EP 2 346 486 B1

**Figure 6**

**Figure 7**

Figure 8

**Figure 9**

**Figure 10**

**Figure 11**

EP 2 346 486 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0019974 A
- EP 1600461 A
- WO 2007099997 A
- EP 0866808 A
- EP 0939771 A
- EP 1076547 A
- EP 0668072 A
- FR 2836378
- WO 02085827 A
- WO 2005105123 A

- FR 2822821
- FR 2857596
- WO 04112741 A
- WO 2006114443 A
- WO 0405005 A
- WO 0121605 A
- WO 2005115421 A
- WO 0121150 A
- WO 9847479 A

**Littérature non-brevet citée dans la description**

- **ADATTO M.A. ; DEPREZ P.** *Journal of Cosmetic Dermatology,* Avril 2003, vol. 2 (2), 61-67